# EUROPEAN PATENT SPECIFICATION

(11) **EP 4 739 206 B1**
(45) Date of publication and mention of the grant of the patent: **28.10.2026**
(21) Application number: 25746988.2
(22) Date of filing: 25.06.2025
(51) Int. Cl.: A61B 5/0537, A61B 5/11, A61B 5/00

(54) **SYSTEM AND METHOD FOR CONTINUOUSLY MEASURING A POSTURE-COMPENSATED HYDRATION OF A SUBJECT**
SYSTEM UND VERFAHREN ZUR KONTINUIERLICHEN MESSUNG EINER HALTUNGSKOMPENSIERTEN HYDRIERUNG EINER PERSON
SYSTÈME ET PROCÉDÉ DE MESURE CONTINUE D'UNE HYDRATATION POUR POSTURE COMPENSÉE D'UN SUJET

(30) Priority: 27.06.2024 NO 20240697
(43) Date of publication of application: 13.05.2026
(73) Proprietor: MODE SENSORS AS, 7030 Trondheim (NO)
(72) Inventor: SÆTHER, Terje, 7050 Trondheim (NO); NORHEIM, Jørn, 7022 Trondheim (NO); CECCHINATO, Daniele, 7039 Trondheim (NO); AAS, Sigve Nyvik, 7054 Ranheim (NO); BREMNES, Frida, 7049 Trondheim (NO); HOGSTAD, Signe, 7018 Trondheim (NO); NORE, Anders, 7550 Hommelvik (NO)
(74) Representative: Bryn Aarflot AS
(86) International application number: PCT/NO2025/050119
(87) International publication number: WO 2026/005615

(56) References cited:
- EP-A1- 4 299 001
- US-A1- 2008 039 700
- US-A1- 2017 340 238
- US-A1- 2019 104 989
- DUTT ABHILASH GURU ET AL: "Wearable bioimpedance for continuous and context-aware clinical monitoring", 2020 42ND ANNUAL INTERNATIONAL CONFERENCE OF THE IEEE ENGINEERING IN MEDICINE & BIOLOGY SOCIETY (EMBC), IEEE, 20 July 2020 (2020-07-20), pages 3985 - 3988, XP033815360, [retrieved on 20200824], DOI: 10.1109/EMBC44109.2020.9175298
- GUILLERMO MEDRANO ET AL: "Model-based correction of the influence of body position on continuous segmental and hand-to-foot bioimpedance measurements", MEDICAL & BIOLOGICAL ENGINEERING & COMPUTING, SPRINGER, BERLIN, DE, vol. 48, no. 6, 20 April 2010 (2010-04-20), pages 531 - 541, XP019835505, ISSN: 1741-0444

## Description

### Background

Bioimpedance is a technique that measures the resistance of biological tissue through small electrical currents, providing valuable data on physiological attributes of a subject such as fluid status. Patch devices can be used to measure said bioimpedance and provide health and fitness monitoring. A particularly useful application for bioimpedance measuring is to monitor hydration levels of a subject to maintain a balanced hydration and prevent imbalanced hydration such as underhydration, dehydration, overhydration and hyperhydration.

Posture influences a distribution of fluid within the body. Since bioimpedance measurements rely on the principle that the body's tissues conduct electrical currents differently based on their composition and hydration levels, posture can significantly impact the accuracy of hydration measurements. When a subject changes posture, particularly from lying down to standing or vice versa, there are significant shifts in fluid distribution throughout the body which can distort bioimpedance derived hydration monitoring.

Most current hydration measurement systems require a subject to maintain a consistent posture during measurements such that the bioimpedance measurements are accurate and reproducible. Variations in posture between measurement sessions in these systems can lead to inconsistencies, potentially resulting in inaccurate assessments of the subject's fluid status.

Some bioimpedance hydration measuring systems may receive information about the subject's posture from sensors such as accelerometers or cameras. Said posture detection can be used to set parameters in data acquisition in a static manner.

Some systems that measure bioimpedance may be designed for calibration to determine appropriate parameters for acquiring bioimpedance signals tailored to a subject. Calibration results may be stored in the system's memory, allowing retrieval of suitable parameters based on different postures. These systems are not suitable for monitoring subjects with irregular posture patterns.

Problems in the above systems occur when ongoing, continuous, accurate hydration monitoring is desired for subjects with irregular posture patterns, and for which static parameter calibration is inadequate. It is an object of the present invention to address some of the above-described limitations in the previous hydration monitoring systems.

DUTT A G et al., Wearable bioimpedance for continuous and context-aware clinical monitoring, 2020 42nd Annual International Conference of the IEEE Engineering in Medicine & Biology Society (EMBC), pp 3985 - 3988 teaches bioimpedance monitoring provides a non-invasive, safe and affordable opportunity to monitor total body water for a wide range of clinical applications. However, the measurement is susceptible to variations in posture and movement. Existing devices do not account for these variations and are therefore unsuitable to perform continuous measurements to depict trend changes. We developed a wearable bioimpedance monitoring system with embedded real-time posture detection using a distributed accelerometer network. We tested the device on 14 healthy volunteers following a standardized protocol of posture change and evaluated the agreement with a commercial device. The impedance showed a high correlation (r>0.98), a bias of -4.5 Ω, and limits of agreement of -30 and 21 Ω. Context-awareness was achieved with an accuracy of 94.6% by classifying data from two accelerometers placed at the upper and lower leg. The calculated current consumption of the system was as low as 10 mA during continuous measurement operation, suggesting that the system can be used for continuous measurements over multiple days without charging. The proposed motion-aware design will enable the measurement of relevant bioimpedance parameters over long periods and support informed clinical decision making.

US2019104989A1 discloses a method of monitoring hydration including obtaining biological data for a given period of time, wherein the biological data includes measurements of one or more biological indicators; converting the biological data into a baseline value; obtaining real-time biological data from one or more biological sensors; performing a pre-processing analysis of the real-time biological data; comparing the real-time biological data with baseline value to create a hydration index.

EP4299001A1 discloses a device comprising a bioimpedance unit configured to periodically or irregularly sense a bioimpedance of at least one part of the body of the user via electrodes that touch a portion of the body around a limb or/and a portion of the body between the limbs of the user, a processing unit that determines at least one physiological parameter of the user based on the bioimpedance and anthropometric data of the user and estimates, based on the at least one physiological parameter, a discrepancy between a current physiological state of the user and the homeostatic state of the user, its origin and compensation status; and an alert module that issues a message concerning the current physiological state of the user and an a recommendation for effective way of treatment of a homeostatic state decompensation.

### Summary of the Invention

According to a first aspect of the invention, there is provided a system for continuously measuring a posture-compensated hydration of a subject, the system comprising: an accelerometer for measuring raw posture data of the subject at incremental time intervals; an electrode arrangement for measuring a bioimpedance signal; at least one processor; and storage media with instructions that, when executed on the processor, perform the tasks of: at a first time interval: a) deriving a continuous single posture dependent signal from the raw posture data; b) applying a transformation filter to the single posture dependent signal to shift the posture signal to align with the time domain of the bioimpedance signal, said transformation filter imitating a response to posture change of a body; c) normalising the filtered posture signal using posture normalisation parameters; d) normalising the bioimpedance signal using bioimpedance normalisation parameters; e) subtracting the normalised filtered posture signal from the normalised impedance signal; and f) renormalising the resulting subtracted signal using the bioimpedance normalisation parameters to find a continuous posture-compensated hydration signal; and repeating steps a) to f) for each subsequent incremental time interval; wherein, at the first time interval, posture normalisation parameters are based on predetermined average parameters for a selected demographic in which the subject is represented; and
wherein, at each subsequent incremental time interval, posture normalisation parameters are adjusted based on each new measurement.

The accelerometer may be of a two-angle type configured to measure three vector components, and wherein storage media with instructions that, when executed on the processor , perform the tasks of deriving a continuous single posture dependent signal by mapping the three vector components of the raw posture data.

At least the accelerometer and the electrode arrangement of the system may be implemented into a skin-adhering patch device.

The patch device may be in data communication with the at least one processor and the storage media with instructions, said processor and said storage media with instructions supported externally to the patch device.

The storage media may further comprise instructions that, when executed on the processor, perform the tasks of: normalising the filtered posture signal and the bioimpedance signal to scale to one another using a standard score normalisation.

The storage media may further comprise instructions that, when executed on the processor, perform the tasks of: optimising the bioimpedance normalisation parameters via a feedback loop from the renormalised bioimpedance signal.

The transformation filter may be adaptive to learn the particular fluid equilibrate delay times after different posture changes and movements over time for a specific monitored subject to learn a subject-dependent time constant.

The transformation filter may employ machine learning to learn the particular fluid equilibrate delay times after different posture changes and movements over time for a specific monitored subject to learn a subject-dependent time constant.

The duration of the incremental time intervals may be between 1 seconds and 180 seconds.

According to a second aspect of the invention there is provided a method for continuously measuring a posture-compensated hydration of a subject, the method comprising: simultaneously and continuously measuring: raw posture data of the subject using an accelerometer at incremental time intervals; and a bioimpedance signal of the subject at incremental time intervals; deriving a continuous single posture dependent signal using the raw posture data; applying a transformation filter to the single posture dependent signal to shift the posture signal to align with the time domain of the bioimpedance signal, said transformation filter imitating a response to posture change of a body; normalising the filtered posture signal using posture normalisation parameters based on the raw posture data, said posture normalisation parameters adjusted with each new incremental time interval measurement; normalising the bioimpedance signal using bioimpedance normalisation parameters based on the bioimpedance signal, said normalisation parameters adjusted with each new measurement; subtracting the normalised filtered posture signal from the normalised impedance signal; and renormalising the resulting subtracted signal using the bioimpedance normalisation parameters to find a continuous posture-compensated hydration signal; wherein, at the first time interval t0, posture normalisation parameters are based on predetermined average parameters for a selected demographic in which the subject is represented and, at each subsequent incremental time interval, posture normalisation parameters are adjusted based on each new measurement.

Normalising the filtered posture signal and the bioimpedance signal to scale to one another may comprise using a standard score normalisation.

The method may further comprise optimising the bioimpedance normalisation parameters via a feedback loop from the renormalised bioimpedance signal.

The accelerometer may measure on two angles which is combined into three vector components for the three dimensions of space and the continuous single posture dependent signal is derived by mapping the three vector components.

The duration of the incremental time intervals may be between 1 seconds and 180 seconds.

The method may further comprise employing machine learning to learn the particular fluid equilibrate delay times after different posture changes and movements over time for a specific monitored subject to learn a subject-dependent time constant of the transformation filter.

According to a third aspect of the invention there is provided computer readable medium comprising instructions that, when executed on a processor, perform the method of the second aspect of the invention.

### Brief Description of the Drawings

Fig. 1 is a flow chart of a method for continuously measuring a posture-compensated hydration of a subject;
Fig. 2 shows an example accelerometer for use in a sensor device for carrying out the method of the invention;
Fig. 3 shows a graph having an idealized impedance signal, an idealised filtered posture signal and a known hydration;
Fig. 4 shows a graph of the idealised filtered posture signal and the idealized impedance signal normalised to a common scale;
Fig. 5a shows a recovered hydration signal overlaid against the known hydration signal;
Fig. 5b shows a recovered hydration signal overlaid against the known hydration signal wherein the recovered hydration signal has been recovered using "on-the-fly" normalisation parameters;
Fig. 6 shows a variance for both bioimpedance and posture stabilizing over time;
Fig. 7a shows an example single posture dependent signal taken from a real experiment according to the method of the invention;
Fig. 7b shows an example continuous bioimpedance signal taken from a real experiment according to the method of the invention;
Fig. 7c is a segment of the posture signal from Fig. 7a overlaid with a corresponding segment of the bioimpedance signal from Fig. 7b;
Fig. 8a shows the bioimpedance signal and posture signal after filtering overlaid on the same plot;
Fig. 8b shows overlaid signals of normalised bioimpedance and normalised filtered posture;
Fig. 9 shows an example system according to the invention for continuously measuring a posture-compensation hydration of a subject;
Fig. 10a shows a top graph of posture and bioimpedance signal of a subject, and a corresponding bottom graph showing a first hydration signal calculated according to the method of the invention and a second hydration signal according to a method of the invention for a subject showing a regular diurnal rhythm and no substantial change in hydration;
Fig. 10b shows a top graph of posture and bioimpedance signal of a subject, and a corresponding bottom graph showing a first hydration signal calculated according to the method of the invention and a second hydration signal according to a method of the invention for a subject showing an irregular diurnal rhythm and a dehydration event; and
Fig. 10c shows a top graph of posture and bioimpedance signal of a subject, and a corresponding bottom graph showing a first hydration signal calculated according to the method of the invention and a second hydration signal according to a method of the invention for a subject showing an irregular diurnal rhythm and no substantial change in hydration.

### Detailed Description

In order to monitor whether a subject is hydrated over a duration, an averaging of the subject's bioimpedance can be calculated and further bioimpedance measurements can be measured against predetermined limits determined from the bioimpedance average.

This technique requires that a subject's average bioimpedance is regular. However, this technique is not suitable for subjects with irregular posture patterns, for example if a subject lies down for several days. Posture compensation of the bioimpedance signal is required.

Thus, the method and system of the invention aim to remove a majority of the bioimpedance resulting from a subject's posture, and which comprises the biggest effect of the bioimpedance signal. Although pressure on the patch from stretch resulting from the patch movement and temperature change have some small effect these are considered negligible compared to the posture effect.

In a supine position, fluid tends to redistribute more evenly throughout the body, resulting in a relatively consistent impedance profile. However, when a subject transitions to an upright posture, such as standing or sitting, gravitational forces cause fluids to accumulate in certain areas, such as the lower extremities. This redistribution of fluid leads to changes in the body's overall impedance characteristics.

For instance, in the standing position, there is typically an increase in fluid volume in the legs and a decrease in fluid volume in the upper body. This shift in fluid distribution alters the electrical conductivity properties of the tissues, thereby affecting the impedance measurements. As a result, bioimpedance measurements taken in different postures may yield varying results, potentially leading to inaccuracies in the assessment of parameters such as body composition, hydration status, and fluid balance. More specifically, and for a patch sensor device positioned on an upper back of a subject, when the subject is lying down, an overall measured bioimpedance will fall because more fluid accumulates where the patch is disposed, and when the subject is standing up, fluid will start to flow downwards to the feet due to gravity. There will, therefore, be less fluid accumulating adjacent where the patch is disposed, and the impedance will increase.

Understanding the impact of posture on bioimpedance measurements is therefore important for interpreting bioimpedance results accurately and ensuring the validity of assessments related to fluid status and hydration of a monitored subject. Integrating posture detection and adjustment mechanisms into bioimpedance measurement systems can help mitigate the effects of posture variations, enhancing the precision and reliability of the obtained data.

The invention provides a method and system for posture-compensated hydration monitoring on a continuous basis to account for "on-the-fly" changes in statistical parameters relating to a subject's posture and bioimpedance to provide accurate hydration tracking.

Users of the method and system of the invention herein, and for which accurate hydration tracking is useful include people at risk of imbalanced hydration such as underhydration, dehydration, overhydration and hyperhydration. Some non-limiting examples of such demographic groups include athletes, pilots, the elderly and people in care. The invention is particularly suitable for subjects at risk of dehydration with irregular posture rhythm over time. The method of the invention compensates for delayed effects on fluid movement after posture changes of the subject which cannot be easily calibrated for due to their irregularity.

Athletes are at risk of dehydration due to the intense levels of exertion during competing. Replenishing fluid lost through sweat can be challenging. Pilots try to stay as dehydrated as possible without impairing their skills since opportunities for urination during flight are limited. Since loss of concentration or even consciousness can be fatal for a pilot, and a symptom of dehydration, accurate hydration monitoring is important. Fluid shifting in the pilot's body may be affected by g-forces of the aircraft in flight, instead of posture, an embodiment of the invention may provide g-force compensated bioimpedance tracking for accurate hydration monitoring.

The elderly are a demographic who are both at risk of dehydration events and are likely to have irregular diurnal patterns. They are at risk of dehydration since their sense of thirst can be reduced. They are likely to have irregular diurnal patterns due to the elderly requiring more frequent urination, often in much shorter intervals than a standard sleep duration, and potential long term resting due to health problems. Thus, this demographic is particularly suitable for use of the present invention.

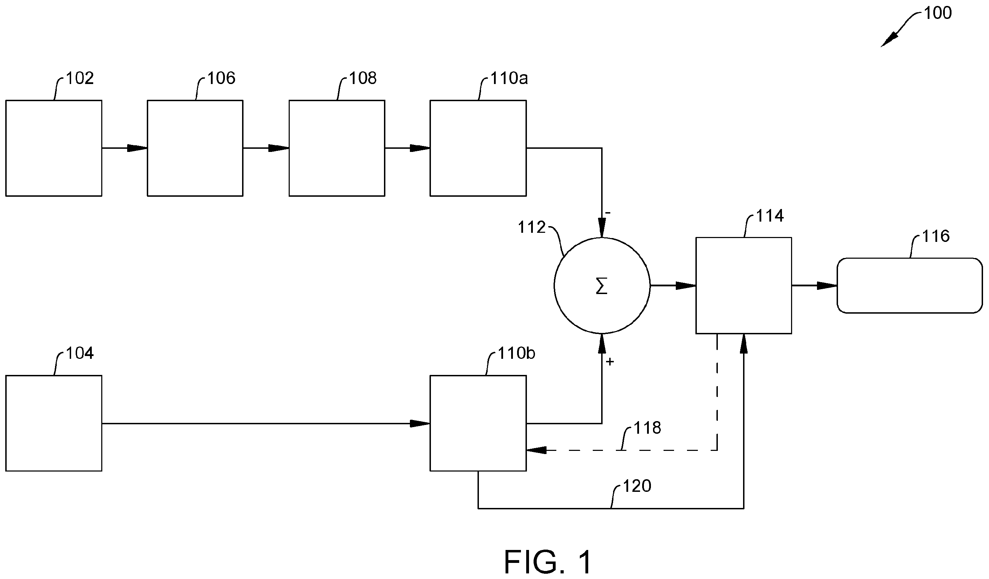

Figure 1 shows a flowchart of a method 100 of the invention. The method 100 is a continuous loop process over incremental time intervals tₙ. Over time the continuous loop process continuously measures a posture-compensated hydration of a subject.

The incremental time intervals tₙ are preferably every 30 seconds but may be a duration between 30 seconds and 60 seconds, such as every 40 seconds, 45 seconds, 50 seconds etc. Preferably, each of the plurality of time intervals are equal in duration. After a subject changes posture, for example from a supine to an upright position, fluid in the body of the subject displaces under gravity. Fluid movement in a subject typically takes some time after a corresponding posture change, and so ongoing measurements at incremental time intervals having a duration between 30 and 60 seconds is effectively continuous in practical terms.

At 102, raw posture data is measured every predetermined incremental time interval tₙ on an ongoing basis to provide continuous raw posture data.

In a preferred embodiment the raw posture data is measured by an accelerometer. Preferably the accelerometer is of the type that measures on two angles which can be combined into three vector components for the three dimensions of space to provide a three-dimensional model of the subject's posture. However, simpler up and down measurements of the subject from the supine to the upright position and vice versa are adequate for carrying out the method 100.

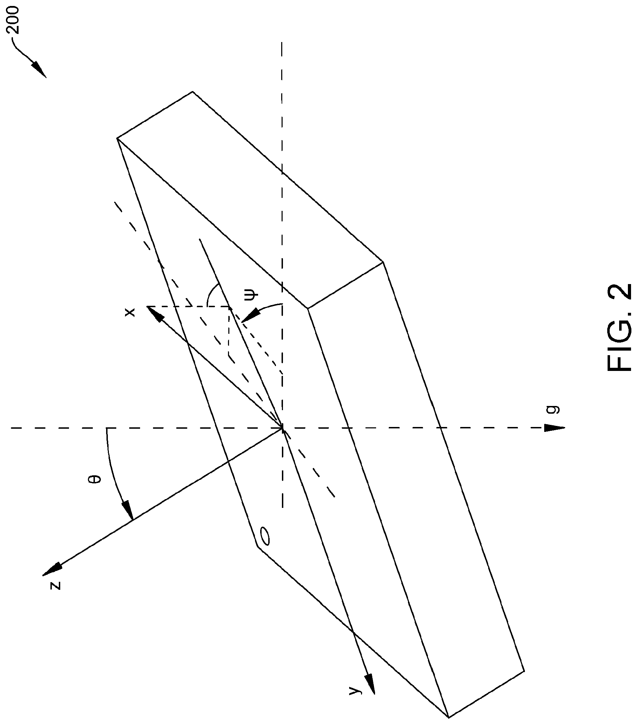

Figure 2 shows an example accelerometer 200 for use in the method 100. **In an** example, the accelerometer is applied vertically to the upper body so that the relation between an angle *θ* and bioimpedance is assumed to be linear.

For the accelerometer 200, the magnitudes of the acceleration vectors can be calculated as follows: ${a}_{x} = g sin θ cos φ$ Equation 1 ${a}_{y} = - g sin θ sin φ$ Equation 2 ${a}_{z} = g cos θ$ Equation 3 $\frac{{a}_{y}}{{a}_{x}} = - tan φ$ Equation 4 wherein *aₓ* is the acceleration in the x axis, *a_{y}* is the acceleration in the y-axis, *a_{z}* is the acceleration in the z-axis, *g* is the Earth's gravity, *θ* is the angle between the vector of the Earth's gravity and the z-axis and *φ* is the angle between the orthogonal of the vector of the Earth's gravity and the x-axis.

A benefit of using an accelerometer to measure the subject's posture is that such a device can be compact have dimensions of a few square millimetres, and require simple electronics, for incorporation into a patch-type sensor device,.

With further reference to Figure 1, at 106, a continuous single posture dependent signal is derived, for example by mapping the three vector components of the raw posture data measured by the accelerometer using multiple linear regression such as that shown in Equation 5 below. $Y = {β}_{0} + {β}_{1} {X}_{1} + {β}_{2} {X}_{2} + {β}_{3} {X}_{3} + ε$ Equation 5 wherein *β*₀ is an intercept, *X*₁ = *aₓ*, *X*₂ *= a_{y}*, *X*₃ *= a_{z}*, *β*₁ is the coefficient for predictor *X*₁, *β*₂ is the coefficient for predictor *X*₂, *β*₃ is the coefficient for predictor *X*₃, and *ε* is a random error term.

This mapping is achieved by gathering initial accelerometer data, formulating the multiple linear regression model Y, fitting the multiple linear regression model to the collected data, and using the fitted model to predict the posture-dependent signal for new accelerometer data.

More specifically, accelerometer data, which includes measurements of three vector components (e.g., *aₓ*, *a_{y}*, and *a_{z}*) that represent posture is gathered. Each component ( *aₓ, a_{y}*, and *a_{z}*) captures a different aspect of the posture. Next, a response variable that represents the posture-dependent signal is identified. This could be a derived measure such as angle of tilt, overall stability score, or another relevant metric that quantifies posture. Then the multiple linear regression model Y is formulated where the response variable (i.e. posture-dependent signal) is predicted by the three vector components. The formulated multiple linear regression model Y is fit to the collected data and the coefficients (*β*₀, *β*₁, *β*₂, *β*₃, and *ε* are estimated which minimize error in predicting the associated response variable. The fitted model is used to predict the posture-dependent signal for new accelerometer data. By inputting new values of X₁ = *aₓ*, *X*₂ *= a_{y}*, and *X*₃ = *a_{z}* into the regression equation, the model can generate a corresponding posture signal.

At 104, a continuous bioimpedance signal of the subject is measured at incremental time intervals on an ongoing basis simultaneously to the measuring og the continuous raw posture data.

At 108, a transformation filter is applied to the single posture dependent signal, said transformation filter imitating a response to posture change of a body. Some body fluid flows immediately or rapidly after a posture change. For example, within seconds, within around 10 seconds, or within around 30 seconds. However, some body fluid takes several hours to equilibrate after a posture change, such as up to three hours. For example, when a subject moves from a supine position to an upright position it may take up to three hours for all the fluid going from the back to equilibrate towards the subject's feet.

Fluid moving from one side of the subject's body to the other side equilibrates more quickly than fluid moving up and down. This gives rise to different time constants involved in different movements when the subject changes posture. Furthermore, fluid equilibrate delay after posture change is subject dependent and can vary significantly from person to person. The transformation filter in step 108 is therefore adaptive and employs machine learning to learn the particular fluid equilibrate delay times after different posture changes and movements over time for a specific monitored subject. The method uses measured data over time to learn the time delay parameters for the posture dependent signal transformation filter.

More specifically, the transformation filter in step 108 is developed by comparing the bioimpedance signal shape and posture signal shape to derive a time constant of the transformation filter. Simple filter types that can be used for the transformation filter include simple filters with a single time constant such as Infinite Impulse Response (IIR) filters. **IIR** filters use previous output values as input, which means they implement a feedback system. This allows them to maintain an internal state and produce an output that depends on previous outputs and current and past inputs.

Thus, since fluid shift delays in the body are movement dependent and can vary from person to person, the transformation filter in step 108 is adaptive and employs machine learning to learn the time constant. One person may have a fluid shift of ±5%, another person ±10%. In a particular example, a dialysis patient may have a fluid shift up to ±20%. The time for the transformation filter to optimise the time constant depends on the posture movements of the subject. At least one instance of fluid shift from moving for the supine to the upright position is required for the model to adapt to the subject.

At 110a, the filtered posture signal is normalised using normalisation parameters based on the raw posture data from the monitoring start time t₀ to the current time interval in the monitoring period tₙ. The posture signal normalisation parameters are adjusted with each new incremental time interval measurement.

At 110b, the bioimpedance signal is normalised using normalisation parameters based on the bioimpedance signal from time t₀ to tₙ. The bioimpedance signal normalisation parameters are adjusted with each new incremental time interval measurement.

The normalisation transformation steps 110a, 110b facilitate comparison of the filtered posture signal and the bioimpedance signal which have different variables, units and scales. The normalisation transformation steps 110a, 110b are especially useful for the machine learning aspect of the method which performs better and converges faster as a result of the similarly scaled data. Following steps 110a and 110b, a comparison between bioimpedance and posture can be made since they are scaled to the same level.

In an example, normalising the filtered posture signal and the bioimpedance signal to scale to one another is achieved using a standard score normalisation. Standard score normalization is a statistical method used to normalize data in a dataset. Values in the dataset are adjusted so that they have a mean of zero and a standard deviation of one. After normalisation of the filtered posture signal and the bioimpedance signal are scaled to the same level.

At 112, the normalised filtered posture signal is subtracted from the normalised impedance signal.

At 114, the resulting subtracted signal is renormalised using the normalisation parameters of the bioimpedance signal to find a continuous posture-compensated hydration signal, 116.

As mentioned above, the method 100 is a continuous loop process for incremental time intervals tₙ such that, over time, the continuous loop process continuously measures a posture-compensated hydration of a subject, the first initial time intervals before the process havs had time to accumulate real data on a real subject, normalisation parameters are based on predetermined average parameters for a selected demographic in which the subject is represented. However, over time, and with each incremental data measurement acquisition, normalisation parameters are adjusted to incorporate each new measurement. For example, if using a standard score normalisation, for each measurement performed, new statistical parameters such as exponential weighted mean and variance are calculated and become the new normalisation parameters. This can be defined as "on-the-fly" updating of normalisation parameters.

Using incremental calculation of the normalisation parameters enables accurate, up-to-date statistics without the overhead of recalculating with each new piece of data. This provides significant performance improvements in the method for continuous posture-compensated hydration monitoring dealing with large volumes of data and requiring real-time processing. More specifically, there is a statistical mean and also statistical variation, so the normalisation steps 110a, 110b begin with mean parameters for the demographic which represents the monitored subject. Preferably, these mean parameters are pre-calculated and stored in a memory of the sensor device used in the method and are acquired using machine learning to extract a statistical parameter set from a plurality of test sensor device experiments. Since the statistical parameter for normalisation differ from person to person, the statistical parameter set is then refined by a further machine learning model using data retrieved from the particular sensor device employed in the method 100 to learn the parameter for that particular person being monitored.

The process is further optimisable by adjusting the normalisation of the continuously measured bioimpedance signal via a feedback loop from the renormalised bioimpedance signal.

Preferably the method includes monitoring the recovered posture-compensated hydration signal against predetermined threshold percentage variations. The method may provide an alert or warning when it is calculated that the hydration signal is outside of the predetermined threshold indicating a dehydration event.

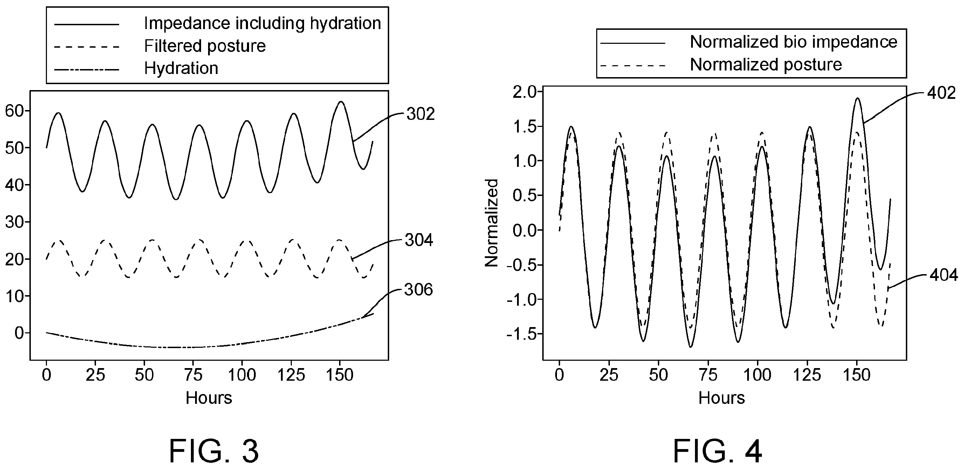

Figures 3 to 6 show graphical representations of stages of the method 100 using idealized signals and a known hydration signal to demonstrate the viability of the concept of the invention.

Figure 3 shows a graph having three signals: impedance 302, filtered posture 304 and hydration 306. To demonstrate the concept, the aim is to subtract impedance 302 from the posture 304 and find the hydration 306.

As per step 110a and 110b of the method 100, both the bioimpedance and the filtered posture are normalised to a common scale. This results in a normalised bioimpedance signal 402 and a normalised posture signal 404 as shown in the graph of Figure 4.

As per step 112 of the method, the normalised filter posture signal 404 is subtracted from the normalised bioimpedance signal 402, after which the resulting remaining signal is renormalised to provide a recovered hydration signal.

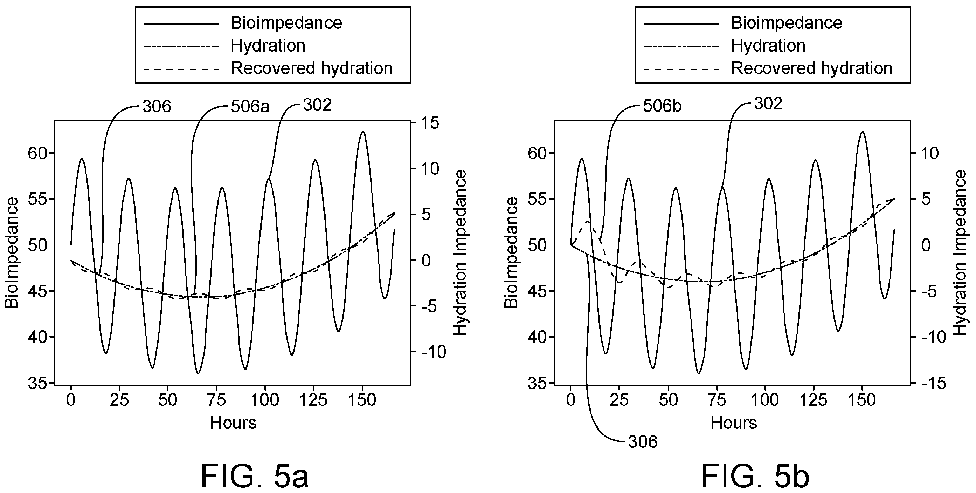

Figure 5a shows the recovered hydration signal 506a overlaid for comparison against the known hydration signal 306. As demonstrated by Figure 5a, the recovered hydration signal 506a deviates slightly from the known hydration signal 306. The recovered hydration signal 506a demonstrates a shallow sinusoidal pattern around the known hydration signal 306 line. Thus, the method 100 does not recover the true hydration signal with 100% accuracy, however, the accuracy of the recovered signal by the method 100 is suitable for its required purpose. The error between the recovered signal by method 100 and the true hydration signal is due to the normalised bioimpedance which is compromised by the hydration signal.

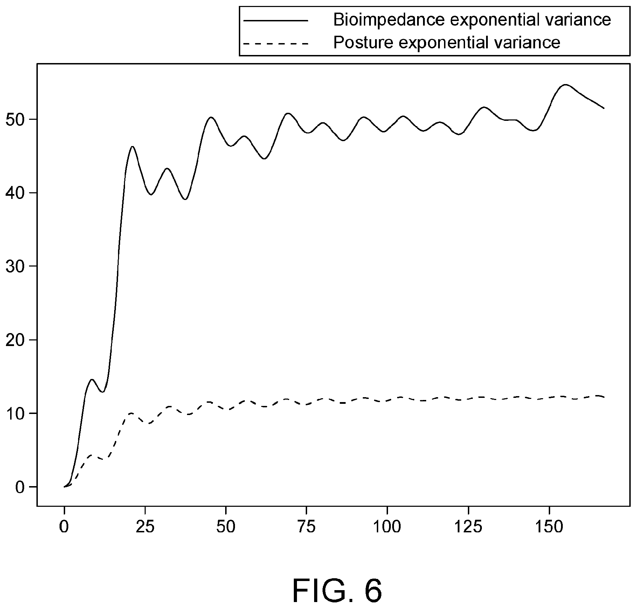

Figure 5b shows a similar graph to the graph of Figure 5a wherein the recovered hydration signal has been recovered using "on-the-fly" normalisation parameters. As the statistical values and hence the normalisation changes over time, the recovered hydration signal is less smooth than the first example where the statistical properties were known. However, this method is more resilient if the statistical properties of the signal itself change over time. Figure 6 shows how the variance for both bioimpedance and posture stabilises over time.

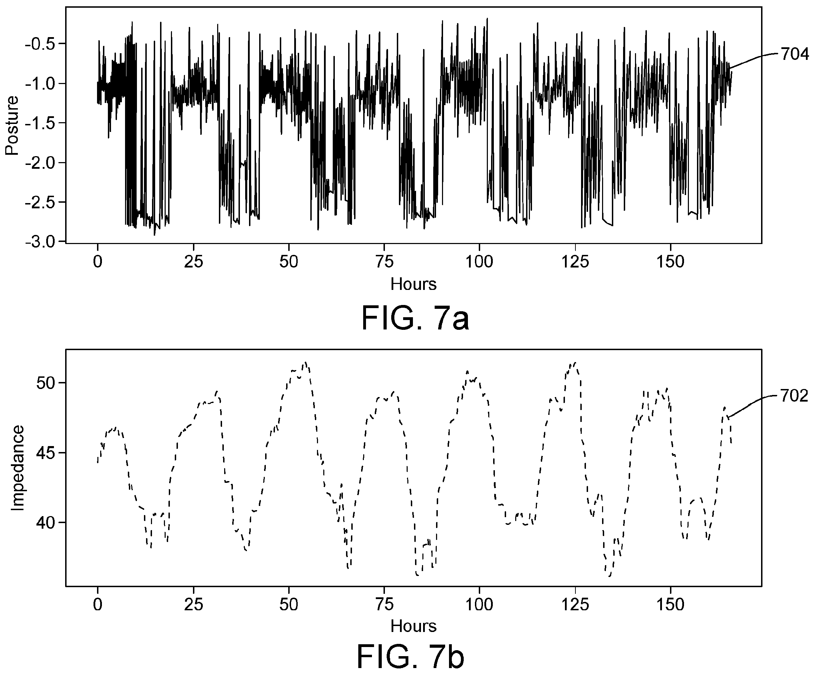

Figures 7a to 8b show graphical representations of the steps 102 to 110 of the method 100 carried out on a real subject over a period of 175 hours.

Figure 7a shows an example single posture dependent signal 704 taken from a real experiment of the above method 100 and using an associated system applied to an elderly person, and thus shows an example outcome of step 106 of process 100, wherein a continuous single posture dependent signal is derived. This example posture-dependent signal 704 was derived by an accelerometer measuring on two angles *θ*, *φ* which were then subsequently combined into three vector components for the three dimensions of space as per equations 1 to 4 above. A mapping of combination of the two angles *θ*, *φ*, provides the single posture dependent signal as is shown in the Figure 7a. In this example the accelerometer was applied vertically to the upper body of the subject so that the relationship between the angle *θ* in Equation 1 to Equation 4 and bioimpedance was assumed to be linear. The posture dependent signal 704 of Figure 7a is substantially regular, having a cyclic pattern within a 24-hour period. In this example, high posture values, between -1.5 and -0.5, represent when the user is in an upright position, such as standing up and shows the subject's posture during day-time hours. In contrast, low posture values, between -2.5 and -3 represent when the subject is in a supine position during the nighttime. The posture dependent signal 704 shows some amount of activity during the night.

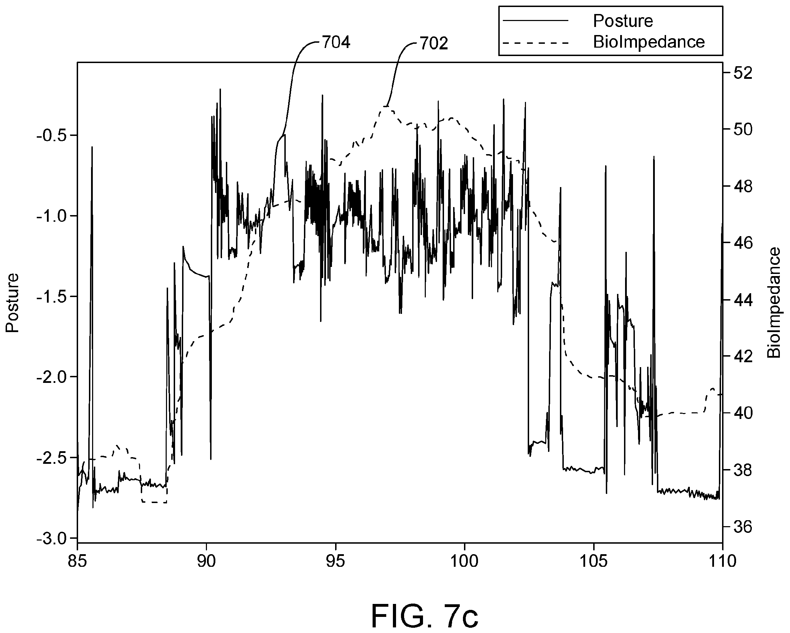

Figure 7b shows an example continuous bioimpedance signal 702 of the same real subject as in Figure 7a and during the same time period of 175 hours. Figure 7b is an example outcome of step 104 of the process 100 wherein a continuous bioimpedance signal of the subject is measured (at incremental time intervals) on an ongoing basis simultaneously to the continuous raw posture data. The continuous bioimpedance signal 702 shows a similar semi-regular sinusoidal cyclic pattern as the posture signal 704. However, it can be seen that the scales between impedance and posture are significantly different. In the example of Figure 7b, the posture signal ranges from -3.0 to -0.5 radians and the impedance signal ranges from 35 to 55 ohms. Typical values for the impedance signal range are from 35 to 100 ohms. Thus, normalisation of the signals is preformed to converge them to a common scale in preparation for the subtraction step 112 of method 100. In Figure 7c, a segment of the posture signal 704 is overlaid with a corresponding segment of the bioimpedance signal 702. Not only are the scales different between the two measurements but the dynamic response to posture change is also different. Posture change is immediate whereas a resultant fluid shift in the subject's body takes time to complete. This delayed effect can take up to several hours. As described above, the time delay of the resultant fluid shift allows for a measurement time interval of between 30 seconds and 60 seconds to be considered continuous.

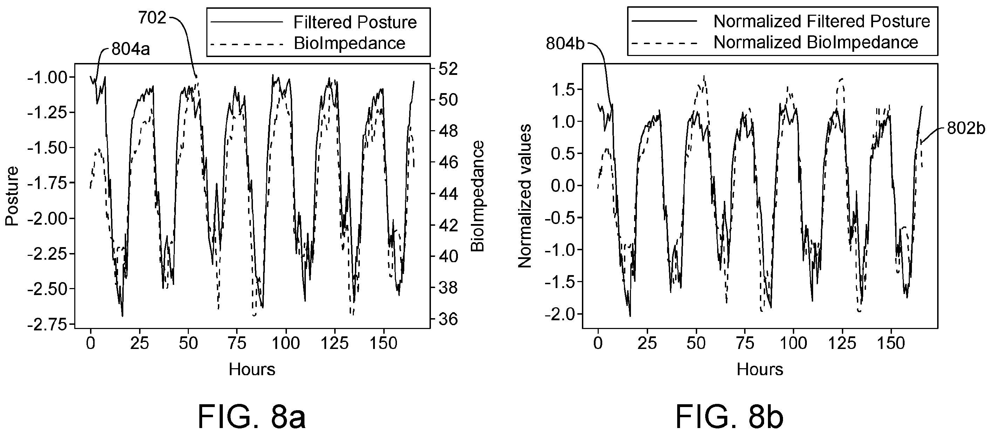

As a result of the different dynamic behaviours of posture and bioimpedance, a filter which mimics the body's response to posture changes is applied to the measured posture. Figure 8a shows the bioimpedance signal 702 and posture signal 804 after application of the filter which mimics the body response to posture changes overlaid on the same plot. Figure 8a still shows two different scales since it is prior to the signal normalisation steps 110a, 110b.

Figure 8b shows overlaid signals of normalised bioimpedance 802b and normalised filtered posture 804b.

As well as major changes in a subject's posture such as moving from a supine position to a standing position, minor changes in a subject's posture can also affect fluid movement. These minor changes include body orientation and body inclination. In the example of body orientation, the monitored subject may rotate from a first side to a second side in the supine position causing fluid movement from one side of the body to the other. Thus, in an example a rotation and orientation of the subject is measured alongside the major posture changes of upright and supine. The rotation and orientation of the subject may be measured, for example using the two-angle accelerometer of Figure 2 or a gyroscope, and combined with measurements from the accelerometers to provide comprehensive data on body posture and movement. In the example of body inclination, the monitored subject may be sitting in a reclined position. An inclinometer may be used to measure the angle of tilt of the subject in relation to the Earth's gravity, making them suitable for assessing the inclination of the body or its parts in static positions. A magnetometer may also be used in conjunction with the accelerometer and one or more of the gyroscope and inclinometer to enhance position and orientation detection.

According to another aspect of the invention, there is provided a system for continuously measuring a posture-compensation hydration of a subject. The system has a sensor device, the sensor device having an electronics module for continuously measuring bioimpedance of a subject and an accelerometer. The system further has a memory storage device, and a processor. The accelerometer is suitable for measuring a posture of the subject at predetermined intervals on an ongoing basis. Preferably the accelerometer is an accelerometer of the type described with reference to Figure 2 which measures on two angles which can be combined into three vector components for the three dimensions of space. The memory storage device comprises computer readable media comprising instructions that, when executed on the processor perform the tasks of continuously measuring a posture-compensated hydration of a subject. In particular, the system is suitable for implementing the method 100 described above. The memory storage device comprising instructions and the processor may be integrated with, or external to the sensor device. The memory storage device comprising instructions and the processor may be located on a separate computer or on a cloud-based system, the computer or cloud-based system data communicable with the sensor device.

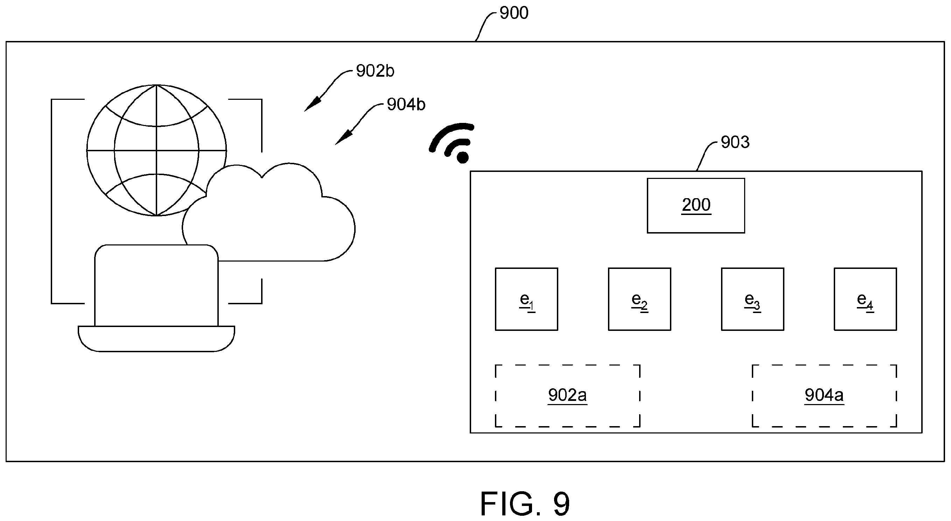

Figure 9 is an example system 900 according to the invention for continuously measuring a posture-compensation hydration of a subject. The system 900 includes a patch sensor device 903 which is configured to be adhered to a subject's skin. Preferably sensor device 903 is configured to be located on an upper back of the subject between the shoulder blades. The sensor device 903 has four electrodes e1, e2, e3, e4, an accelerometer 200, and a memory storage device 902a, and a processor 904a. The sensor device 903 may further comprise a rechargeable battery (not shown). The sensor device 903 is preferably alight weight, body mountable, and flexible patch. The sensor device 903 preferably makes time resolved electrical resistance and reactance measurements over a wide range of frequencies such as the sensor device described in patent NO20201071. In an example, the sensor device 903 has one or more further position measurement devices such as a gyroscope, inclinometer and magnetometer to measure comprehensive data on body posture and movement. The sensor device 903 is in data communication with a memory storage device 902b and a processor 904b located on a cloud-based platform or separate computer. The memory storage device 902b comprises computer readable medium comprising instructions that when executed on the processor 904b perform the task of the method 100 of the invention described herein.

In an example of the invention, the method includes adjustment parameters on the bioimpedance signal for where the sensor device is disposed on the subject's body. For example, whether the sensor device is placed on the left side or the right side of the subject's body. This will impact the bioimpedance signal with respect to a subject's sleeping position. For example, if the subject is a left side sleeper and the sensor device is disposed on the left side of the subject's body, the impedance signal will be lower than if the sensor device is disposed on the right side.

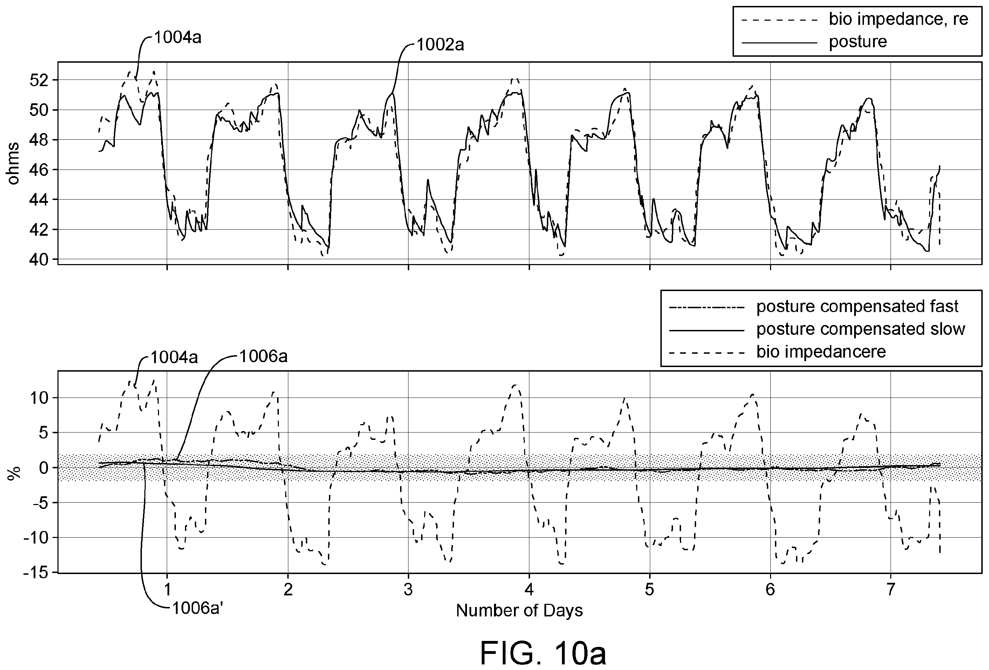

Figures 10a to 10c show three different posture-hydration scenarios measured according to the method and associated system of the invention. In figures 10a to 10c the top graph shows measured bioimpedance and measured posture, and the bottom graph shows the measured bioimpedance and calculated hydration according to a low pass filter. There is provided a first low pass filter with a short time constant and second low pass filter with a long time constant. The first low pass filter has a high cut off frequency. The second low pass filter has a low cut off frequency. In this way, rapid changes are filtered away. The utilisation of the short time constant as in the first low pass filter and the long time constant as in the second low pass filter provides filtering at two different time scales. The signals were acquired according to the above-described method 100 over a monitoring period of 7 days.

In Figure 10a, with reference to the top graph, a substantially sinusoidal posture signal 1002a was measured. This indicates that the subject being monitored had a substantially regular diurnal rhythm over the monitoring period. The x-axis shows the monitoring period of 7 days. The posture signal 1002a shows substantially sinusoidal peaks and troughs patterns over a 24-hour period indicating night-time periods in a generally reclined/recumbent position (sleeping) and day-time periods in a generally upright position (awakeness). The posture signal is high for around 16 hours and low for around 8 hours every day.

With reference to the bottom graph of Figure 10a, a first hydration signal 1006a is shown. The first hydration signal 1006a is a posture compensated bioimpedance recovered according to the method 100 of the invention and associated system 900 herein. A second hydration signal 1006a' is also shown in the bottom graph of Figure 10a. The second hydration signal 1006' is also a posture compensated bioimpedance recovered according to the method 100 of the invention and associated system 900 herein. In the instance of the first hydration signal 1006a, the hydration signal is recovered using a first low pass filter. The signal recovered using a filter with a short time constant provides results faster, within a time frame of a few hours. In the instance of the second hydration signal 1006a', the hydration signal is recovered using a filter with a long time constant. The signal recovered using a second low pass filter provides results over a much longer time delay of around 24 hours, however, accuracy of the results is greater than using the filter with a short time constant. There is a tradeoff between response time and accuracy. A slow filter (long time constant) will average more samples and thus remove more of the short term changes in the signal.

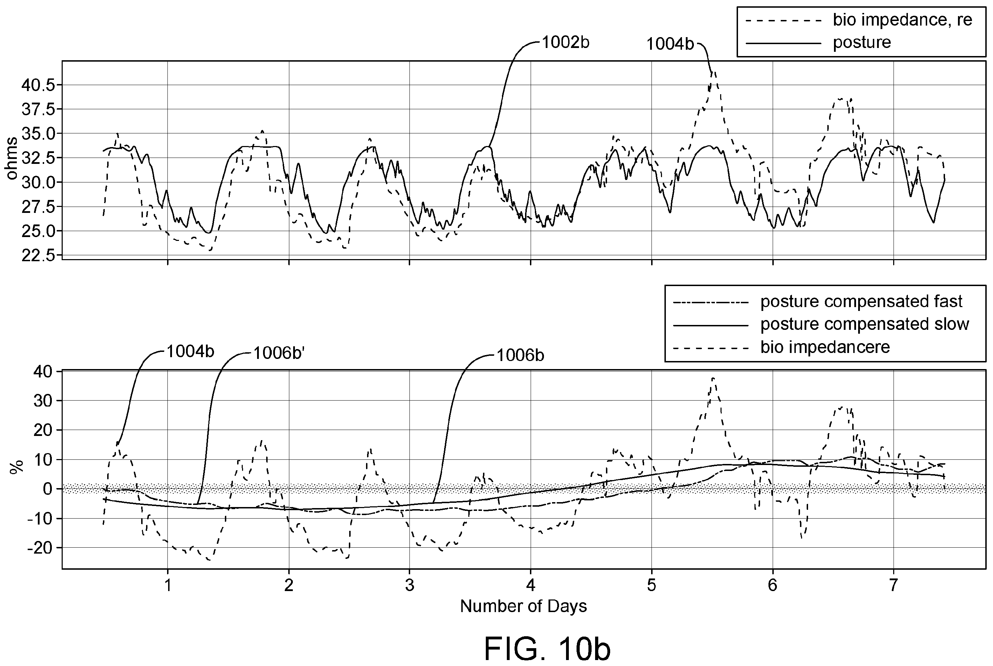

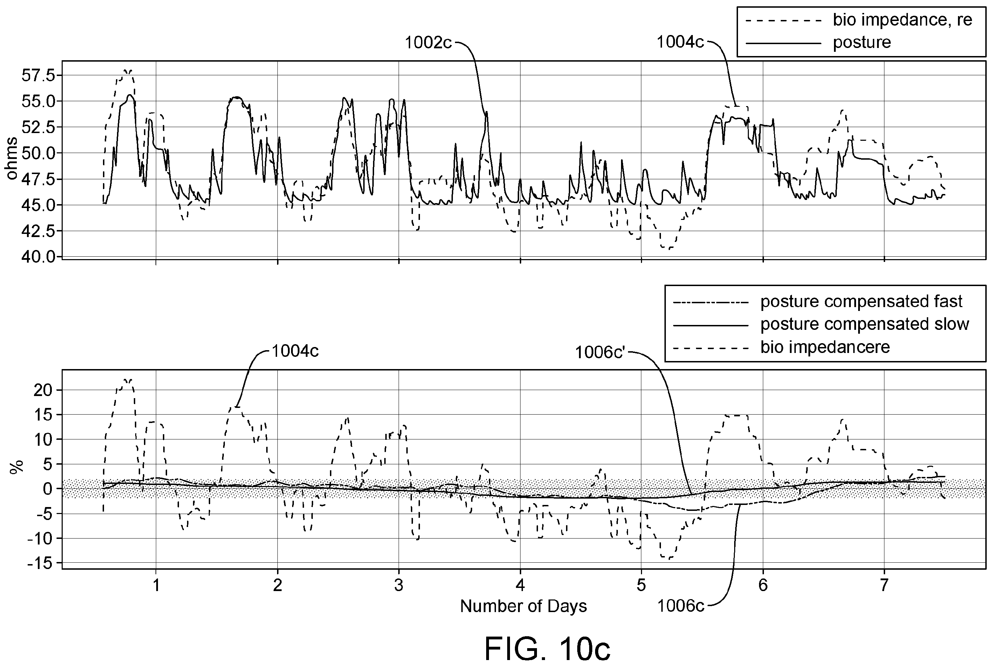

The shaded area of the graph shows a percentage hydration deviation of ±2%, i.e. a "hydration zone". In both the first and second hydration signals are substantially horizontal and remain within the shaded area, and thus can be considered stable hydration of the subject over the monitoring period, i.e. the subject remains inside the shaded area and remains within the "hydrated zone". Since the monitored subject in Figure 10a has a very regular diurnal pattern, continuous posture compensation does not greatly improve the accuracy of the recovered hydration signal over simply taking an average hydration signal over one 24-hour period. However, this is not the case in the scenarios represented by Figures 10b and 10c as explained below.

In Figure 10b, with reference to the top graph, the posture signal 1002b is less regular in comparison to Figure 10a. Between day 2.5 and day 5 the posture signal is particularly irregular. This indicates that the monitored subject had an irregular diurnal rhythm over the monitoring period. The posture signal 1002a indicates night-time periods of wakefulness. The bioimpedance signal 1004b is also less regular in comparison to Figure 10a, having a strong irregularity around day 4 to 5. Since the subject's diurnal rhythm is irregular, simply using the bioimpedance signal does not give an accurate measurement of the subject's hydration. Taking an average posture calibration of the bioimpedance also does not provide accurate hydration measuring. In order to ascertain how much of the irregularity in the bioimpedance signal is a result of the subject's irregular posture, the above-described method 100 to calculate posture compensated bioimpedance is performed to provide the bottom graph of figure 10b.

With reference to the bottom graph of Figure 10b, a first hydration signal 1006b and a second hydration signal 1006b' are shown. A positive value represents dehydration, while a negative value represents hydration.The shaded area of ±2% may represent a threshold deviation inside of which a deviation change is deemed acceptable. Both the first and second hydration signals fall outside of the shaded "hydration zone" and thus signals 1006b, 1006b' indicate the subject has experienced an imbalanced hydration during the monitoring period.

In Figure 10c, with reference to the top graph, the posture signal 1002c is irregular over the 7-day monitoring period and the subject has a very irregular diurnal rhythm. Between day 3 and day 5.5 the posture signal indicates a long period of reclination. This may have been a period of sickness experienced by the subject. The bioimpedance signal 1004c is also irregular. Similarly to the signals retrieved in Figure 10b, since the subject's diurnal rhythm is so irregular, simply using the bioimpedance signal does not give an accurate measurement of the subject's hydration. Taking an average posture calibration of the bioimpedance also does not provide accurate hydration measuring. In order to ascertain how much of the irregularity in the bioimpedance signal is a result of the subject's irregular posture, the above-described method 100 to calculate posture compensated bioimpedance is performed to provide the bottom graph of Figure 10c. The smaller peaks in the posture signal from day 3 to day 5.5 can for example represent the subject moving from one side to the other side, sitting halfway or recline in the bed a bit and then laying down. The method 100 of using up and down posture changes still provides good results for this posture signal. Additional minor posture movement tracking, for example by a two-angle accelerometer or a gyroscope, may lead to an improved hydration signal.

With reference to the bottom graph of Figure 10c, first and second hydration signals 1006c, 1006c' are shown. In the case of the first hydration signals, the signals remain within the shaded area, and thus can be considered to represent stable hydration of the subject over the monitoring period. A positive value represents dehydration, while a negative value represents hydration.The shaded area of ±2% may represent a threshold deviation inside of which a deviation change is deemed acceptable. However, in the case of the second hydration signal, the signal drops below the -2% threshold on day 5, suggesting an imbalanced hydration in the subject during the monitoring period.

The present invention is particularly useful for providing ongoing hydration monitoring for subjects with irregular diurnal patterns. More particularly, the invention is particularly useful for tracking the hydration of elderly people in the nursing home, or in their own homes.

The present invention may also aid tracking hydration levels in pilots using the method and sensor system described above for continuous and "on-the-fly" compensation of gravitational forces due to the acceleration experienced by pilots during rapid changes in velocity or direction of movement. The sensor system may be configured to alert the pilot when their hydration drops below a predetermined hydration level threshold.

Having described preferred examples of the invention it will be apparent to those skilled in the art that other embodiments incorporating the invention may be used. These and other examples of the invention illustrated above are intended by way of example only and the actual scope of the invention is to be determined from the appended claims.

## Claims

1. A system (900) for continuously measuring a posture-compensated hydration of a subject, the system comprising:
an accelerometer (200) for measuring raw posture data of the subject at incremental time intervals;
an electrode arrangement (e₁-e₄) for measuring a bioimpedance signal
at least one processor (904a); and
storage media (902a) with instructions that, when executed on the processor perform the tasks of:
at a first time interval:
a) deriving a continuous single posture dependent signal from the raw posture data;
b) applying a transformation filter to the single posture dependent signal to shift the posture signal to align with the time domain of the bioimpedance signal, said transformation filter imitating a response to posture change of a body of the subject;
c) normalising the filtered posture signal using posture normalisation parameters;
d) normalising the bioimpedance signal using bioimpedance normalisation parameters;
e) subtracting the normalised filtered posture signal from the normalised impedance signal; and
f) renormalising the resulting subtracted signal using the bioimpedance normalisation parameters to find a continuous posture-compensated hydration signal; and
repeating steps a) to f) for each subsequent incremental time interval;
wherein, at the first time interval, posture normalisation parameters are based on predetermined average parameters for a selected demographic in which the subject is represented; and
wherein, at each subsequent incremental time interval, posture normalisation parameters are adjusted based on each new measurement.

2. The system of claim 1, wherein the accelerometer is of a two-angle type configured to measure three vector components, and wherein the storage media comprises instructions that, when executed on the processor, perform the tasks of deriving a continuous single posture dependent signal by mapping the three vector components of the raw posture data.

3. The system of claim 1 or claim 2, wherein at least the accelerometer and the electrode arrangement of the system are implemented into a skin-adhering patch device.

4. The system of claim 3, wherein the patch device is in data communication with the at least one processor and the storage media with instructions, said processor and said storage media with instructions supported externally to the patch device.

5. The system of any of claims 1 to 4, wherein the storage media further comprises instructions that, when executed on the processor, perform the tasks of: normalising the filtered posture signal and the bioimpedance signal to scale to one another using a standard score normalisation.

6. The system of any preceding claim, wherein the storage media further comprises instructions that, when executed on the processor, perform the tasks of:
optimising the bioimpedance normalisation parameters via a feedback loop from the renormalised bioimpedance signal.

7. The system of any preceding claim, wherein the transformation filter is adaptive to learn the particular fluid equilibrate delay times after different posture changes and movements over time for a specific monitored subject to learn a subject-dependent time constant.

8. The system of claim 7, wherein the transformation filter employs machine learning to learn the particular fluid equilibrate delay times after different posture changes and movements over time for a specific monitored subject to learn a subject-dependent time constant.

9. The system of any preceding claim, wherein the duration of the incremental time intervals is between 1 seconds and 180 seconds.

10. A method (100) for continuously measuring a posture-compensated hydration of a subject, the method comprising:
simultaneously and continuously measuring:
raw posture data of the subject using an accelerometer at incremental time intervals; and
a bioimpedance signal of the subject at incremental time intervals;
deriving a continuous single posture dependent signal using the raw posture data;
applying a transformation filter to the single posture dependent signal to shift the posture signal to align with the time domain of the bioimpedance signal, said transformation filter imitating a response to posture change of a body of the subject;
normalising the filtered posture signal using posture normalisation parameters based on the raw posture data, said posture normalisation parameters adjusted with each new incremental time interval measurement;
normalising the bioimpedance signal using bioimpedance normalisation parameters based on the bioimpedance signal, said normalisation parameters adjusted with each new measurement;
subtracting the normalised filtered posture signal from the normalised impedance signal; and
renormalising the resulting subtracted signal using the bioimpedance normalisation parameters to find a continuous posture-compensated hydration signal;
wherein, at the first time interval t0, posture normalisation parameters are based on predetermined average parameters for a selected demographic in which the subject is represented and, at each subsequent incremental time interval, posture normalisation parameters are adjusted based on each new measurement.

11. The method of claim 10, wherein normalising the filtered posture signal and the bioimpedance signal to scale to one another comprises using a standard score normalisation.

12. The method of claim 10 or claim 11, further comprising
optimising the bioimpedance normalisation parameters via a feedback loop from the renormalised bioimpedance signal.

13. The method of any of claims 10 to 12, wherein the accelerometer measures on two angles which is combined into three vector components for the three dimensions of space and the continuous single posture dependent signal is derived by mapping the three vector components.

14. The method of any of claims 10 to 13, wherein the duration of the incremental time intervals is between 1 seconds and 180 seconds.

15. The method of any of claims 10 to 14, further comprising employing machine learning to learn the particular fluid equilibrate delay times after different posture changes and movements over time for a specific monitored subject to learn a subject-dependent time constant of the transformation filter.

16. Computer readable medium comprising instructions that, when executed on a processor, perform the method of any of claims 10 to 15.

## Patentansprüche

1. System (900) zur kontinuierlichen Messung einer haltungskompensierten Hydratation einer Person, wobei das System umfasst:
einen Beschleunigungsmesser (200) zum Messen von rohen Haltungsdaten der Person in inkrementellen Zeitintervallen;
eine Elektrodenanordnung (e₁-e₄) zum Messen eines Bioimpedanzsignals;
mindestens einen Prozessor (904a); und
Speichermedien (902a) mit Anweisungen, die, wenn sie auf dem Prozessor ausgeführt werden, die Aufgaben durchführen, zum, in einem ersten Zeitintervall:
a) Ableiten eines kontinuierlichen einzigen haltungsabhängigen Signals aus den rohen Haltungsdaten;
b) Anwenden eines Transformationsfilters auf das einzige haltungsabhängige Signal, um das Haltungssignal so zu verschieben, dass es mit der Zeitdomäne des Bioimpedanzsignals ausgerichtet wird, wobei der Transformationsfilter eine Reaktion auf eine Haltungsänderung eines Körpers der Person imitiert;
c) Normalisieren des gefilterten Haltungssignals unter Verwendung von Haltungsnormalisierungsparametern;
d) Normalisieren des Bioimpedanzsignals unter Verwendung von Bioimpedanznormalisierungsparametern;
e) Subtrahieren des normalisierten gefilterten Haltungssignals von dem normalisierten Impedanzsignal; und
f) erneutes Normalisieren des resultierenden subtrahierten Signals unter Verwendung der Bioimpedanznormalisierungsparameter, um ein kontinuierliches haltungskompensiertes Hydratationssignal zu finden; und Wiederholen der Schritte a) bis f) für jedes nachfolgende inkrementelle Zeitintervall;
wobei im ersten Zeitintervall Haltungsnormalisierungsparameter auf vorbestimmten Durchschnittsparametern für eine ausgewählte Bevölkerung basieren, in der die Person vertreten ist; und
wobei in jedem nachfolgenden inkrementellen Zeitintervall Haltungsnormalisierungsparameter basierend auf jeder neuen Messung angepasst werden.

2. System nach Anspruch 1, wobei der Beschleunigungsmesser vom Zweiwinkeltyp ist, der ausgebildet ist, um drei Vektorkomponenten zu messen, und wobei das Speichermedium Anweisungen umfasst, die, wenn sie auf dem Prozessor ausgeführt werden, die Aufgaben des Ableitens eines kontinuierlichen haltungsabhängigen Signals durch Zuordnen der drei Vektorkomponenten der rohen Haltungsdaten durchführen.

3. System nach Anspruch 1 oder Anspruch 2, wobei zumindest der Beschleunigungsmesser und die Elektrodenanordnung des Systems in einer an der Haut haftenden Patch-Vorrichtung implementiert sind.

4. System nach Anspruch 3, wobei die Patch-Vorrichtung mit dem mindestens einen Prozessor und dem Speichermedium mit Anweisungen in Datenkommunikation steht, wobei der Prozessor und das Speichermedium mit Anweisungen extern an der Patch-Vorrichtung angebracht sind.

5. System nach einem der Ansprüche 1 bis 4, wobei das Speichermedium weiter Anweisungen umfasst, die, wenn sie auf dem Prozessor ausgeführt werden, die folgenden Aufgaben durchführen: maßstabsgerechtes Normalisieren des gefilterten Haltungssignals und des Bioimpedanzsignals zueinander unter Verwendung einer Standardwerte-Normalisierung.

6. System nach einem vorstehenden Anspruch, wobei das Speichermedium weiter Anweisungen umfasst, die, wenn sie auf dem Prozessor ausgeführt werden, die folgenden Aufgaben durchführen:
Optimieren der Bioimpedanz-Normalisierungsparameter über eine Rückkoppelungsschleife aus dem erneut normalisierten Bioimpedanzsignal.

7. System nach einem vorstehenden Anspruch, wobei der Transformationsfilter anpassungsfähig ist, um die spezifischen Fluidausgleichsverzögerungszeiten nach unterschiedlichen Haltungsänderungen und Bewegungen im Laufe der Zeit für eine bestimmte überwachte Person zu erlernen, um eine personenabhängige Zeitkonstante zu erlernen.

8. System nach Anspruch 7, wobei der Transformationsfilter Maschinenlernen einsetzt, um die spezifischen Fluidausgleichsverzögerungszeiten nach unterschiedlichen Haltungsänderungen und Bewegungen im Laufe der Zeit für eine bestimmte überwachte Person zu erlernen, um eine personenabhängige Zeitkonstante zu erlernen.

9. System nach einem vorstehenden Anspruch, wobei die Dauer der inkrementellen Zeitintervalle zwischen 1 Sekunde und 180 Sekunden beträgt.

10. Verfahren (100) zur kontinuierlichen Messung einer haltungskompensierten Hydratation einer Person, wobei das Verfahren umfasst:
gleichzeitiges und kontinuierliches Messen:
von rohen Haltungsdaten der Person unter Verwendung eines Beschleunigungsmessers in inkrementellen Zeitintervallen; und
eines Bioimpedanzsignals der Person in inkrementellen Zeitintervallen;
Ableiten eines kontinuierlichen einzigen haltungsabhängigen Signals unter Verwendung der rohen Haltungsdaten;
Anwenden eines Transformationsfilters an das einzige haltungsabhängige Signal, um das Haltungssignal so zu verschieben, dass es mit der Zeitdomäne des Bioimpedanzsignals ausgerichtet wird, wobei der Transformationsfilter eine Reaktion auf eine Haltungsänderung eines Körpers der Person imitiert;
Normalisieren des gefilterten Haltungssignals unter Verwendung von Haltungsnormalisierungsparametern basierend auf den rohen Haltungsdaten, wobei die Haltungsnormalisierungsparameter mit jeder neuen inkrementellen Zeitintervallmessung angepasst werden;
Normalisieren des Bioimpedanzsignals unter Verwendung von Bioimpedanznormalisierungsparametern basierend auf dem Bioimpedanzsignal, wobei die Normalisierungsparameter mit jeder neuen Messung angepasst werden;
Subtrahieren des normalisierten gefilterten Haltungssignals von dem normalisierten Impedanzsignal; und
erneutes Normalisieren des resultierenden subtrahierten Signals unter Verwendung der Bioimpedanznormalisierungsparameter, um ein kontinuierliches haltungskompensiertes Hydratationssignal zu finden;
wobei im ersten Zeitintervall t0 Haltungsnormalisierungsparameter auf vorbestimmten Durchschnittsparametern für eine ausgewählte Bevölkerung basieren, in der die Person vertreten ist, und in jedem nachfolgenden inkrementellen Zeitintervall Haltungsnormalisierungsparameter basierend auf jeder neuen Messung angepasst werden.

11. Verfahren nach Anspruch 10, wobei maßstabsgerechtes Normalisieren des gefilterten Haltungssignals und des Bioimpedanzsignals zueinander Verwenden einer Standardwerte-Normalisierung umfasst.

12. Verfahren nach Anspruch 10 oder Anspruch 11, weiter umfassend
Optimieren der Bioimpedanz-Normalisierungsparameter über eine Rückkoppelungsschleife aus dem erneut normalisierten Bioimpedanzsignal.

13. Verfahren nach einem der Ansprüche 10 bis 12, wobei der Beschleunigungsmesser an zwei Winkeln misst, was in drei Vektorkomponenten für die drei Raumdimensionen kombiniert wird, und das kontinuierliche haltungsabhängige Signal durch Zuordnen der drei Vektorkomponenten abgeleitet wird.

14. Verfahren nach einem der Ansprüche 10 bis 13, wobei die Dauer der inkrementellen Zeitintervalle zwischen 1 Sekunde und 180 Sekunden beträgt.

15. Verfahren nach einem der Ansprüche 10 bis 14, weiter umfassend Einsetzen von Maschinenlernen, um die spezifischen Fluidausgleichsverzögerungszeiten nach unterschiedlichen Haltungsänderungen und Bewegungen im Laufe der Zeit für eine bestimmte überwachte Person zu erlernen, um eine personenabhängige Zeitkonstante des Transformationsfilters zu erlernen.

16. Computerlesbares Medium, das Anweisungen umfasst, die, wenn sie auf einem Prozessor ausgeführt werden, das Verfahren nach einem der Ansprüche 10 bis 15 durchführen.

## Revendications

1. Système (900) de mesure continue d'une hydratation compensée par posture d'un sujet, le système comprenant :
un accéléromètre (200) pour mesurer des données de posture brutes du sujet à des intervalles de temps incrémentiels ;
un agencement d'électrodes (e₁-e₄) pour mesurer un signal de bioimpédance ;
au moins un processeur (904a) ; et
des supports de stockage (902a) comportant des instructions qui, lorsqu'elles sont exécutées sur le processeur effectuent les tâches consistant à :
à un premier intervalle de temps :
a) déduire un signal continu unique dépendant de la posture à partir des données de posture brutes ;
b) appliquer un filtre de transformation au signal unique dépendant de la posture afin de décaler le signal de posture pour l'aligner sur le domaine temporel du signal de bioimpédance, ledit filtre de transformation imitant une réponse à un changement de posture d'un corps du sujet ;
c) normaliser le signal de posture filtré à l'aide de paramètres de normalisation de posture ;
d) normaliser le signal de bioimpédance à l'aide de paramètres de normalisation de bioimpédance ;
e) soustraire le signal de posture filtré normalisé du signal d'impédance normalisé ; et
f) renormaliser le signal soustrait obtenu à l'aide des paramètres de normalisation de bioimpédance pour trouver un signal continu d'hydratation compensée par posture ; et
répéter les étapes a) à f) pour chaque intervalle de temps incrémentiel suivant ;
dans lequel, au premier intervalle de temps, les paramètres de normalisation de posture sont basés sur des paramètres moyens prédéterminés pour un groupe démographique sélectionné dans lequel le sujet est représenté ; et
dans lequel, à chaque intervalle de temps incrémentiel suivant, les paramètres de normalisation de posture sont ajustés sur la base de chaque nouvelle mesure.

2. Système selon la revendication 1, dans lequel l'accéléromètre est de type à deux angles configuré pour mesurer trois composantes vectorielles, et dans lequel les supports de stockage comprennent des instructions qui, lorsqu'elles sont exécutées sur le processeur, effectuent les tâches consistant à déduire un signal continu unique dépendant de la posture en mappant les trois composantes vectorielles des données de posture brutes.

3. Système selon la revendication 1 ou la revendication 2, dans lequel au moins l'accéléromètre et l'agencement d'électrodes du système sont mis en œuvre dans un dispositif de patch adhérant à la peau.

4. Système selon la revendication 3, dans lequel le dispositif de patch est en communication de données avec l'au moins un processeur et les supports de stockage comportant des instructions, ledit processeur et lesdits supports de stockage comportant des instructions étant situés à l'extérieur du dispositif de patch.

5. Système selon l'une quelconque des revendications 1 à 4, dans lequel les supports de stockage comprennent en outre des instructions qui, lorsqu'elles sont exécutées sur le processeur, effectuent les tâches consistant à : normaliser le signal de posture filtré et le signal de bioimpédance pour qu'ils soient mis à l'échelle l'un par rapport à l'autre à l'aide d'une normalisation par score standard.

6. Système selon une quelconque revendication précédente, dans lequel les supports de stockage comprennent en outre des instructions qui, lorsqu'elles sont exécutées sur le processeur, effectuent les tâches consistant à :
optimiser les paramètres de normalisation de bioimpédance via une boucle de rétroaction à partir du signal de bioimpédance renormalisé.

7. Système selon une quelconque revendication précédente, dans lequel le filtre de transformation est adaptatif pour apprendre les temps de retard d'équilibrage de fluide particuliers après différents changements de posture et mouvements au fil du temps pour un sujet surveillé spécifique afin d'apprendre une constante de temps dépendante du sujet.

8. Système selon la revendication 7, dans lequel le filtre de transformation emploie l'apprentissage automatique pour apprendre les temps de retard d'équilibrage de fluide particuliers après différents changements de posture et mouvements au fil du temps pour un sujet surveillé spécifique afin d'apprendre une constante de temps dépendante du sujet.

9. Système selon une quelconque revendication précédente, dans lequel la durée des intervalles de temps incrémentiels est comprise entre 1 seconde et 180 secondes.

10. Procédé (100) de mesure continue d'une hydratation compensée par posture d'un sujet, le procédé comprenant :
la mesure simultanée et continue :
de données de posture brutes du sujet à l'aide d'un accéléromètre à des intervalles de temps incrémentiels ; et
un signal de bioimpédance du sujet à des intervalles de temps incrémentiels ;
la déduction d'un signal continu unique dépendant de la posture à l'aide des données de posture brutes ;
l'application d'un filtre de transformation au signal unique dépendant de la posture afin de décaler le signal de posture pour l'aligner sur le domaine temporel du signal de bioimpédance, ledit filtre de transformation imitant une réponse à un changement de posture d'un corps du sujet ;
la normalisation du signal de posture filtré à l'aide de paramètres de normalisation de posture basés sur les données de posture brutes, lesdits paramètres de normalisation de posture étant ajustés à chaque nouvelle mesure d'intervalle de temps incrémentiel ;
la normalisation du signal de bioimpédance à l'aide de paramètres de normalisation de bioimpédance basés sur le signal de bioimpédance, lesdits paramètres de normalisation étant ajustés à chaque nouvelle mesure ;
la soustraction du signal de posture filtré normalisé du signal d'impédance normalisé ; et
la renormalisation du signal soustrait obtenu à l'aide des paramètres de normalisation de bioimpédance pour trouver un signal continu d'hydratation composée par posture ;
dans lequel, au premier intervalle de temps t0, les paramètres de normalisation de posture sont basés sur des paramètres moyens prédéterminés pour un groupe démographique sélectionné dans lequel le sujet est représenté et, à chaque intervalle de temps incrémentiel suivant, les paramètres de normalisation de posture sont ajustés sur la base de chaque nouvelle mesure.

11. Procédé selon la revendication 10, dans lequel la normalisation du signal de posture filtré et du signal de bioimpédance pour qu'ils soient mis à l'échelle l'un par rapport à l'autre comprend l'utilisation d'une normalisation par score standard.

12. Procédé selon la revendication 10 ou revendication 11, comprenant en outre
l'optimisation des paramètres de normalisation de bioimpédance via une boucle de rétroaction à partir du signal de bioimpédance renormalisé.

13. Procédé selon l'une quelconque des revendications 10 à 12, dans lequel l'accéléromètre mesure selon deux angles qui sont combinés en trois composantes vectorielles pour les trois dimensions de l'espace et le signal continu unique dépendant de la posture est déduit en mappant les trois composantes vectorielles.

14. Procédé selon l'une quelconque des revendications 10 à 13, dans lequel la durée des intervalles de temps incrémentiels est comprise entre 1 seconde et 180 secondes.

15. Procédé selon l'une quelconque des revendications 10 à 14, comprenant en outre l'emploi de l'apprentissage automatique pour apprendre les temps de retard d'équilibrage de fluide particulier après différents changements de posture et mouvements au fil du temps pour un sujet surveillé spécifique afin d'apprendre une constante de temps dépendante du sujet du filtre de transformation.

16. Support lisible par ordinateur comprenant des instructions qui, lorsqu'elles sont exécutées sur un processeur, réalisent le procédé selon l'une quelconque des revendications 10 à 15.
